Europäisches Patentamt

European Patent Office

Office européen des brevets

(11) Veröffentlichungsnummer: **0 490 221 B1**

(12) **EUROPÄISCHE PATENTSCHRIFT**

(45) Veröffentlichungstag der Patentschrift: **09.08.95**

(21) Anmeldenummer: **91120710.8**

(22) Anmeldetag: **03.12.91**

(51) Int. Cl.⁶: **C07C  41/28**, C07C 43/15

(54) Verfahren zur Herstellung von ungesättigten Ethern.

(30) Priorität: **14.12.90 DE 4039950**

(43) Veröffentlichungstag der Anmeldung:
**17.06.92 Patentblatt  92/25**

(45) Bekanntmachung des Hinweises auf die
Patenterteilung:
**09.08.95 Patentblatt  95/32**

(84) Benannte Vertragsstaaten:
**AT BE CH DE DK ES FR GB IT LI LU NL SE**

(56) Entgegenhaltungen:
**EP-A- 0 217 089**          **EP-A- 0 299 286**
**EP-A- 0 327 985**          **DD-A- 247 669**
**DE-A- 3 224 033**          **GB-A- 2 091 259**
**US-A- 3 023 250**

**CHEMICAL ABSTRACTS, vol. 54, no. 22, 25.
November 1960, Columbus, Ohio, US; abstract no. 24452g, L.A. YANOVSKAYA ET AL.
'Chemistry of acetals. I. A general method of
synthesis of tetraethyl acetals of beta-dicarbonyl compounds'**

(73) Patentinhaber: **HOECHST AKTIENGESELL-
SCHAFT**

**D-65926 Frankfurt am Main (DE)**

(72) Erfinder: **Weber, Jürgen, Dr. Dipl.-Chem.
Bunsenstrasse 17
W-4200 Oberhausen (DE)**
Erfinder: **Lappe, Peter, Dr. Dipl.-Chem.
Eickenhof 34
W-4220 Dinslaken (DE)**
Erfinder: **Springer, Helmut, Dipl.-Ing.
Borbecker Strasse 19
W-4200 Oberhausen (DE)**

**Beschreibung**

Die vorliegende Erfindung betrifft ein Verfahren zur Herstellung von Ethern der Formel $R^1\text{-}CR^2 = CH\text{-}O\text{-}R^3$, worin $R^1$ für einen Rest mit 1 bis 6 Kohlenstoffatomen, $R^2$ für Wasserstoff oder einen Rest mit 1 bis 2 Kohlenstoffatomen und $R^3$ für einen Rest mit 1 bis 6 Kohlenstoffatomen steht. Ether der vorstehend genannten Art besitzen aufgrund ihrer Eigenschaften für eine Reihe technischer Anwendungen Bedeutung. Sie lassen sich als wichtige Bausteine und Ausgangsstoffe für die Synthese von Spezialchemikalien, Pharmazeutika, Naturstoffen und Pflanzenschutzmitteln verwenden.

Die DE-PS 28 44 635 beschreibt ein Verfahren zur Herstellung von 2-Propyl-pent-4-en-1-al, wobei das aus n-Valeraldehyd und 2 Mol Allylalkohol hergestellte Diacetal in Gegenwart eines sauren Katalysators, beispielsweise p-Toluolsulfonsäure, gespalten und der intermediär gebildete Ether thermisch umgelagert wird.

Die US-PS 30 23 250 betrifft die Herstellung halogensubstituierter, ungesättigter Ether durch Spaltung entsprechender 1,1-Di-(2-Halogenalkoxy)-alkane in Gegenwart starker Mineralsäuren oder aromatischer Sulfonsäuren, die unter den Reaktionsbedingungen nicht flüchtig sind. Dem aus der Spaltungsreaktion resultierenden Reaktionsgemisch setzt man innerhalb einer Destillationskolonne eine relativ niedrigsiedende, Stickstoff enthaltende Base, beispielsweise ein Amin, zu. Die Zugabe der Stickstoff enthaltenden Base dient zur Neutralisation von bei der Spaltung des halogenhaltigen Diacetals entstehenden sauren Verbindungen.

L.A. Yanovskaya et al. beschreiben in Otdel. Khim. Nauk 1960, 1246 bis 1253 (Chem. Abstr. 54, 24452g (1960)) ein Verfahren zur Herstellung von ungesättigten Ethern, wobei ein entsprechendes Aldehyddiacetal in Gegenwart eines aus p-Toluolsulfonsäure und Chinolin bestehenden Katalysators unter Abspaltung von Alkohol zu dem ungesättigten Ether umgesetzt wird. Die erzielten Ausbeuten liegen in Abhängigkeit von dem herzustellenden ungesättigten Ether zwischen 40 und 70 %.

Es besteht demzufolge ein Bedarf an einem Verfahren, das einerseits nur einen geringen Aufwand zu seiner Durchführung erfordert und sich andererseits nicht nur auf die Herstellung einiger weniger, spezieller ungesättigter Ether beschränkt, sondern eine Vielzahl ungesättigter Ether zugänglich macht. Das Verfahren soll ferner zu einer Verminderung der unerwünschten Nebenprodukte und zugleich zu einer Erhöhung der Wertproduktausbeute führen.

Gelöst wird diese Aufgabe durch ein Verfahren zur Herstellung von Ethern der Formel $R^1\text{-}CH^2 = CH\text{-}O\text{-}R^3$, worin $R^1$ für einen Rest mit 1 bis 6 Kohlenstoffatomen, $R^2$ für Wasserstoff oder einen Rest mit 1 bis 2 Kohlenstoffatomen und $R^3$ für einen Rest mit 1 bis 6 Kohlenstoffatomen steht, dadurch gekennzeichnet, daß ein Diacetal der Formel $R^1\text{-}CHR^2\text{-}CH(\text{-}O\text{-}R^3)_2$ in Gegenwart eins aus einer Säure und einem aliphatischen oder cycloaliphatischen Amin bestehenden Katalysators bei erhöhter Temperatur umgesetzt wird.

$R^1$ steht für einen Rest mit 1 bis 6, insbesondere 1 bis 4, bevorzugt 1 bis 3 Kohlenstoffatomen. Als Beispiele für $R^1$ sind besonders aliphatische geradkettige oder verzweigte Reste zu nennen. $R^1$ steht für einen Methyl-, Ethyl-, n-Propyl-, i-Propyl-, n-Butyl-, i-Butyl-, n-Pentyl-, i-Pentyl-, 2-Methylbutyl-, 3-Methylbutyl-, n-Hexyl-, i-Hexyl-, insbesondere Methyl-, Ethyl-, n-Propyl-, i-Propyl, n-Butyl-, i-Butyl-, bevorzugt Methyl-, Ethyl-, n-Propyl- oder i-Propylrest.

$R^2$ umfaßt Wasserstoff oder einen Rest mit 1 bis 2 Kohlenstoffatomen, insbesondere einen Methyl- oder Ethylrest.

$R^3$ steht für einen Rest mit 1 bis 6, insbesondere 1 bis 4, bevorzugt 1 bis 3 Kohlenstoffatomen. Als Beispiele für $R^3$ sind insbesondere aliphatische geradkettige oder verzweigte Reste, wie ein Methyl-, Ethyl-, n-Propyl-, i-Propyl-, n-Butyl-, i-Butyl-, n-Pentyl-, i-Pentyl-, 2-Methylbutyl-, 3-Methylbutyl-, n-Hexyl-, i-Hexyl-, insbesondere Methyl-, Ethyl-, n-Propyl-, i-Propyl, n-Butyl-, i-Butyl-, bevorzugt Methyl-, Ethyl-, n-Propyl- oder i-Propylrest zu nennen.

Das erfindungsgemäße Verfahren geht von einem Diacetal der Formel $R^1\text{-}CHR^2\text{-}CH(\text{-}O\text{-}R^3)_2$, worin $R^1$, $R^2$ und $R^3$ die vorstehend beschriebene Bedeutung besitzen, als Ausgangsstoff aus. Derartige Diacetale erhält man durch Umsetzung eines Aldehydes mit einem Alkohol, wobei die Aldehydgruppe mit 2 Alkoholmolekülen unter Bildung von Wasser reagiert. Diese Umsetzung wird üblicherweise in Gegenwart saurer Katalysatoren durchgeführt und das sich bildende Reaktionswasser mit Hilfe eines geeigneten Lösungsmittels durch Azeotropdestillation entfernt. Als Katalysator kann eine Mineralsäure, beispielsweise Schwefelsäure oder Phosphorsäure, oder eine aliphatische oder aromatische Sulfonsäure verwendet werden. Als Lösungsmittel eignen sich alle organischen Lösungsmittel, die mit Wasser ein entsprechendes Azeotrop bilden. Hierunter fallen aliphatische Kohlenwasserstoffe, Cyclohexan, Cyclohexanderivate, Toluol und Xylol. In vielen Fällen hat sich Cyclohexan als besonders geeignetes Lösungsmittel erwiesen.

Nach einer anderen Variante setzt man den Aldehyd mit dem Alkohol in Gegenwart des Lösungsmittels und des sauren Katalysators bei relativ niedrigen Temperaturen um, neutralisiert das aufgrund des zugesetzten Lösungsmittels heterogen anfallende Reaktionsgemisch, trennt die üblicherweise als untere Schicht anfallende Wasserphase ab und entfernt anschließend das Lösungsmittel aus der organischen Phase durch Destillation gegebenenfalls unter reduziertem Druck. Der verbleibende Destillationsrückstand, der das gewünschte Diacetal des Aldehyds bereits in hoher Konzentration enthält, kann unmittelbar in das erfindungsgemäße Verfahren eingesetzt werden.

Das Diacetal wird in Gegenwart eines aus einer Säure und einem Amin bestehenden Katalysators umgesetzt. Der Bedarf an Säure ist recht gering. Je Mol Diacetal werden 0,00015 bis 0,008, insbesondere 0,0002 bis 0,006, bevorzugt 0,00025 bis 0,0015 Mol Säure eingesetzt.

Bei Auswahl der Säure sollte darauf geachtet werden, daß sie eine ausreichende Säurestärke besitzt. Die Säure soll einen pK-Wert $\leq$ 2,5, insbesondere $\leq$ 2,2, bevorzugt $\leq$ 2,0 aufweisen.

Geeignete Säuren sind Phosphorsäure sowie deren partiell veresterte Derivate, Schwefelsäure, Schwefelsäurehalbester, aliphatische oder aromatische Sulfonsäuren, insbesondere aromatische Sulfonsäuren. Als gut geeignet haben sich Benzolsulfonsäure und Toluolsulfonsäure, insbesondere p-Toluolsulfonsäure, erwiesen.

Es hat sich als vorteilhaft herausgestellt, das Amin nicht in beliebiger Menge einzusetzen, sondern bestimmte Mengenverhältnisse zu beachten. Das molare Verhältnis von Säure zu Amin soll 1 : 0,7 bis 1 : 3,5, insbesondere 1 : 0,8 bis 1 : 3, bevorzugt 1 : 1 bis 1 : 2 betragen.

Das als Katalysatorbestandteil fungierende Amin soll unter den Reaktionsbedingungen nicht flüchtig sein und demzufolge einen Siedepunkt besitzen, der Wenigstens 10, insbesondere 20, bevorzugt 30 °C oberhalb des Siedepunktes der sich bildenden Reaktionsprodukte liegt. Als Amine lassen sich primäre, sekundäre und/oder tertiäre Amine verwenden.

Als Kriterium für eine Auswahl geeigneter Amine ist die Anzahl aller im Amin vorhandenen Kohlenstoffatome heranzuziehen. Das Amin soll insgesamt 8 bis 30, insbesondere 9 bis 24, bevorzugt 10 bis 20 Kohlenstoffatomen besitzen.

Geeignete Amine sind cycloaliphatische und aliphatische Amine, insbesondere geradkettige und/oder verzweigte aliphatische Amine.

Beispiele für Amine sind n-Octyl-, n-Nonyl-, n-Decyl-, n-Dodecyl-, 2-Ethylhexyl-, i-Nonyl-, 3,5,5-Trimethylhexyl-, Di-n-butyl-, Di-i-butyl-, Diamyl-, Di-n-hexyl-, Di-n-octyl-, Di-2-ethylhexyl-, Di-i-nonyl-, Tri-n-propyl-, Tri-n-butyl-, Tri-n-pentyl-, Tri-n-hexyl-, Tri-n-octyl-, Tri-2-ethylhexyl-, Tri-n-nonyl-, Tri-i-nonyl- und Tri-n-decylamin.

In vielen Fällen haben sich Isononylamin, Diamylamin, Tri-n-butylamin, Di-2-ethylhexylamin und Diisononylamin als Amin bewährt.

Als Amine werden nicht verstanden stickstoffhaltige heterocyclische Verbindungen, beispielsweise aus der Reihe der Pyridine, Pyrimidine, Pyrrole, Pyrazole und deren Derivate.

Zur Durchführung des erfindungsgemäßen Verfahrens setzt man dem Diacetal das aus der Säure und dem Amin bestehende Katalysatorsystem zu, erhitzt unter Vermischen und trennt die sich im Verlaufe der Umsetzung bildenden Reaktionsprodukte, nämlich den abgespaltenen Alkohol und den ungesättigten Ether der Formel $R^1\text{-}CR^2=CH\text{-}O\text{-}R^3$, ab. Das erfindungsgemäße Verfahren läßt sich sowohl diskontinuierlich - beispielsweise unter Verwendung eines Rührwerkbehälters - als auch kontinuierlich - beispielsweise mittels einer Rührkesselkaskade, einer Glockenbodenkolonne, Füllkörperkolonne - durchführen. Die Umsetzung erfolgt in flüssiger Phase, wobei eine Temperatur eingehalten wird, die oberhalb des Siedepunktes der Reaktionsprodukte liegt.

Als Diacetal der Formel $R^1\text{-}CHR^2\text{-}CH(\text{-}O\text{-}R^3)_2$ kommen offenkettige Diacetale, die vorzugsweise zwei gleiche Acetalreste aufweisen, in Betracht. Beispiele für derartige Diacetale sind Dimethylacetale, Diethylacetale, Di-n-propylacetale, Di-n-butylacetale, Di-i-butylacetale, Di-n-pentylacetale, Di-i-pentylacetale, Di-n-hexylacetale und Di-i-hexylacetale von Aldehyden der allgemeinen Formel $R^1\text{-}CHR^2\text{-}CHO$, worin $R^1$ für einen Rest mit 1 bis 6 Kohlenstoffatomen und $R^2$ für Wasserstoff oder einen Rest mit 1 bis 2 Kohlenstoffatomen steht.

Beispiele derartiger Diacetale sind Acetaldehyd-dimethylacetal, Acetaldehyd-diethylacetal, Acetaldehyd-dipropylacetal, Propionaldehyd-dimethylacetal, Propionaldehyd-diethylacetal, Propionaldehyd-dipropylacetal, Propionaldehyd-dibutylacetal, Butyraldehyd-dimethylacetal, Butyraldehyd-diethylacetal, Butyraldehyd-dipropylacetal, Butyraldehyd-dibutylacetal, Butyraldehyd-dipentylacetal, Valeraldehyd-dimethylacetal, Valeraldehyd-diethylacetal, Valeraldehyd-dipropylacetal, Valeraldehyd-dibutylacetal, Valeraldehyd-dipentylacetal, Isovaleraldehyd-dimethylacetal, Isovaleraldehyd-diethylacetal, Isovaleraldehyd-dipropylacetal, Isovaleraldehyd-dibutylacetal, Isovaleraldehyd-dipentylacetal, Hexanal-dimethylacetal, Hexanal-diethylacetal, Hexanal-dipropylacetal, Hexanal-dibutylacetal, Hexanal-dipentylacetal, Hexanal-dihexylacetal, 2-Ethylhexanal-dimethylacetal, 2-Ethylhexanal-diethylacetal, 2-Ethylhexanal-dipropylacetal, 2-Ethylhexanal-dibutylacetal, 2-Ethylhexanal-dipentylacetal und 2-Ethylhexanal-dihexylacetal.

Als gut geeignet haben sich als Diacetal Propanal-dimethylacetal, Propanal-diethylacetal, Propanal-diisopropylacetal, Propanal-di-n-propylacetal, Butanal-dimethylacetal, Butanal-diethylacetal, Butanal-diisopropylacetal, Butanal-di-n-propylacetal und Valeraldehyd-diethylacetal erwiesen.

Die Reaktion wird üblicherweise bei Normaldruck oder geringfügig erhöhtem Druck durchgeführt, es ist aber auch möglich, sie unter reduziertem Druck ablaufen zu lassen. Auf den Zusatz eines Lösungsmittels kann verzichtet werden.

Die Reaktionstemperatur ist in Abhängigkeit vom Druck zu wählen, sie sollte üblicherweise höchstens 220 bis 240°C betragen. In den meisten Fällen läßt sich die Umsetzung bei Temperaturen von höchstens 200, insbesondere 180, bevorzugt 160°C mit gutem Erfolg durchführen. Das aus der Säure und dem Amin bestehende Katalysatorsystem verbleibt nach Abtrennung der sich bildenden Reaktionsprodukte als Rückstand und kann gegebenenfalls erneut als Katalysator in das erfindungsgemäße Verfahren eingesetzt werden.

Die nachfolgenden Beispiele belegen die Erfindung, ohne sie zu begrenzen.

Experimenteller Teil

I. Herstellung der Ausgangsstoffe

(A): Herstellung von Propionaldehyd-diethylacetal

In einem mit Rührer, Tropftrichter, Rückflußkühler und Innenthermometer bestückten 4 l Dreihalskolben werden 553,2 g Ethanol, 900 g Cyclohexan sowie 5,7 g p-Toluolsulfonsäure vorgelegt und innerhalb von 5 Minuten unter Rühren mit 348,6 g Propionaldehyd versetzt. Infolge der spontan einsetzenden Acetalbildung steigt die Innentemperatur bis auf etwa 40°C an. Man läßt 30 Minuten bei 40°C reagieren, stellt anschließend durch Zugabe von Natronlauge (20 %ige wäßrige Lösung) im Reaktionsgemisch, das aus einer unteren Wasserphase und einer oberen organischen Phase besteht, einen pH-Wert von 7,0 ein und trennt die Wasserphase ab.

Zur Entfernung der Leichtsieder destilliert man die organische Phase bei 1013 mbar bis zu einer Sumpftemperatur von 121°C an.

Es verbleiben nach Abtrennung der Leichtsieder 532,2 g Produkt folgender Zusammensetzung (ermittelt durch gaschromatografische Analyse):

0,6 Gew.-% Ethanol
0,1 Gew.-% Propionaldehyd
3,8 Gew.-% Cyclohexan
93,8 Gew.-% Propionaldehyd-diethylacetal
1,7 Gew.-% sonstige Komponenten

(B): Herstellung von n-Butyraldehyd-di-n-butylacetal

In einem mit Rührer, Tropftrichter, Rückflußkühler und Innenthermometer bestückten 4 l Dreihalskolben werden 889,5 g n-Butanol, 1500 g Cyclohexan sowie 5,7 g p-Toluolsulfonsäure vorgelegt und innerhalb von 5 Minuten unter Rühren mit 432,7 g n-Butyraldehyd versetzt. Infolge der spontan einsetzenden Acetalbildung steigt die Innentemperatur bis auf etwa 35°C an. Man läßt 30 Minuten bei 35 bis 40°C reagieren, stellt anschließend durch Zugabe von Natronlauge (20 %ige wäßrige Lösung) im Reaktionsgemisch, das aus einer unteren Wasserphase und einer oberen organischen Phase besteht, einen pH-Wert von 7,0 ein und trennt die Wasserphase ab.

Zur Entfernung der Leichtsieder destilliert man die organische Phase bei 200 mbar bis zu einer Sumpftemperatur von 160°C an.

Es verbleiben nach Abtrennung der Leichtsieder 973,0 g Produkt folgender Zusammensetzung (ermittelt durch gaschromatografische Analyse):

2,3 Gew.-% n-Butanol
0,1 Gew.-% n-Butyraldehyd
0,1 Gew.-% Cyclohexan
95,7 Gew.-% n-Butyraldehyd-di-n-butylacetal
1,8 Gew.-% sonstige Komponenten

(C): Herstellung von 3-Methylbutyraldehyd-di-n-propylacetal

In einen mit Rührer, Tropftrichter, Rückflußkühler und Innenthermometer bestückten 4 l Dreihalskolben werden 841,4 g n-Propanol, 1750 g Cyclohexan sowie 6,7 g p-Toluolsulfonsäure vorgelegt und innerhalb von 5 Minuten unter Rühren mit 602,7 g 3-Methylbutyraldehyd versetzt. Infolge der spontan einsetzenden Acetalbildung steigt die Innentemperatur bis auf etwa 35°C an. Man läßt 30 Minuten bei 35 bis 40°C reagieren, stellt anschließend durch Zugabe von Natronlauge (20 %ige wäßrige Lösung) im Reaktionsgemisch, das aus einer unteren Wasserphase und einer oberen organischen Phase besteht, einen pH-Wert von 7,0 ein und trennt die Wasserphase ab.

Zur Entfernung der Leichtsieder destilliert man die organische Phase bei 200 mbar bis zu einer Sumpftemperatur von 100°C an.

Es verbleiben nach Abtrennung der Leichtsieder 941,5 g Produkt folgender Zusammensetzung (ermittelt durch gaschromatografische Analyse):

4,5 Gew.-% n-Propanol

0,3 Gew.-% 3-Methylbutyraldehyd

0,2 Gew.-% Cyclohexan

91,9 Gew.-% 3-Methylbutyraldehyd-di-n-propylacetal

3,1 Gew.-% sonstige Komponenten

(D): Herstellung von 2-Ethylbutyraldehyd-dimethylacetal

In einem mit Rührer, Tropftrichter, Rückflußkühler und Innenthermometer bestückten 4 l Dreihalskolben werden 417,3 g Methanol, 1625 g Cyclohexan sowie 6,2 g p-Toluolsulfonsäure vorgelegt und innerhalb von 5 Minuten unter Rühren mit 651,3 g 2-Ethylbutyraldehyd versetzt. Infolge der spontan einsetzenden Acetalbildung steigt die Innentemperatur bis auf etwa 32°C an. Man läßt 30 Minuten bei 40°C reagieren, stellt anschließend durch Zugabe von Natronlauge (20 %ige wäßrige Lösung) im Reaktionsgemisch, das aus einer unteren Wasserphase und einer oberen organischen Phase besteht, einen pH-Wert von 7,0 ein und trennt die Wasserphase ab.

Zur Entfernung der Leichtsieder destilliert man die organische Phase bei 200 mbar bis zu einer Sumpftemperatur von 105°C an.

Es verbleiben nach Abtrennung der Leichtsieder 760,7 g Produkt folgender Zusammensetzung (ermittelt durch gaschromatografische Analyse):

0,1 Gew.-% Cyclohexan

0,3 Gew.-% 2-Ethylbutyraldehyd

97,9 Gew.-% 2-Ethylbutyraldehyd-dimethylacetal

1,7 Gew.-% sonstige Komponenten

II. Herstellung der $\alpha,\beta$-ungesättigten Ether

IIa) Herstellung von Ethyl-1-propenylether

Beispiel 1

In einem mit einer Kolonne (24 theoretische Böden) bestückten 2 l Rundkolben werden 800 g Propionaldehyd-diethylacetal (hergestellt wie unter (A) beschrieben), 0,57 g p-Toluolsulfonsäure und 1,13 g Di-2-ethylhexylamin vorgelegt und erhitzt. Man destilliert die infolge der Acetalspaltung freiwerdenden Reaktionsprodukte (Ethanol und Ethyl-1-propenylether) unter einem Rücklaufverhältnis von 3 : 1 und einem Druck von 1013 mbar ab. Die Temperatur am Kopf der Kolonne beträgt 67 bis 70°C. Die Destillation wird beendet, sobald eine Sumpftemperatur von 220°C erreicht wird. Man erhält 775,4 g Destillat. Die Ausbeute an Ethyl-1-propenylether beträgt 98,2 % der Theorie, bezogen auf eingesetztes Acetal.

Vergleichsbeispiel 1

Man arbeitet wie in Beispiel 1 angegeben, jedoch ohne Zusatz von Di-2-ethylhexylamin. Man erhält 748,5 g Destillat.

Die Ausbeute an Ethyl-1-propenylether beträgt 34,0 % der Theorie, bezogen auf eingesetztes Acetal.

Beispiel 2

Man arbeitet wie in Beispiel 1 angegeben, jedoch unter Zusatz von 0,28 g p-Toluolsulfonsäure und 0,57 g Di-2-ethylhexylamin, und erhält 760,5 g Destillat.

Die Ausbeute an Ethyl-1-propenylether beträgt 95,9 % der Theorie, bezogen auf eingesetztes Acetal.

Beispiel 3

Man arbeitet wie in Beispiel 1 angegeben, jedoch unter Zusatz von 0,28 g p-Toluolsulfonsäure und 1,13 g Di-2-ethylhexylamin, und erhält 754,5 g Destillat.

Die Ausbeute an Ethyl-1-propenylether beträgt 85,5 % der Theorie, bezogen auf eingesetztes Acetal.

Beispiel 4

Man arbeitet wie in Beispiel 1 angegeben, jedoch unter Zusatz von 1,44 g p-Toluolsulfonsäure und 2,84 g Di-2-ethylhexylamin, und erhält 758,5 g Destillat.

Die Ausbeute an Ethyl-1-propenylether beträgt 93,2 % der Theorie, bezogen auf eingesetztes Acetal.

Beispiel 5

Man arbeitet wie in Beispiel 1 angegeben, jedoch unter Zusatz von 5,68 g p-Toluolsulfonsäure und 11,36 g Di-2-ethylhexylamin, und erhält 735,5 g Destillat.

Die Ausbeute an Ethyl-1-propenylether beträgt 89,1 % der Theorie, bezogen auf eingesetztes Acetal.

Beispiel 6

Man arbeitet wie in Beispiel 1 angegeben, jedoch unter Zusatz von 0,57 g p-Toluolsulfonsäure und 0,72 g Di-2-ethylhexylamin, und erhält 772,7 g Destillat.

Die Ausbeute an Ethyl-1-propenylether beträgt 97,8 % der Theorie, bezogen auf eingesetztes Acetal.

Beispiel 7

Man arbeitet wie in Beispiel 1 angegeben, jedoch unter Zusatz von 0,57 g p-Toluolsulfonsäure und 0,58 g Di-2-ethylhexylamin, und erhält 755,4 g Destillat.

Die Ausbeute an Ethyl-1-propenylether beträgt 91,7 % der Theorie, bezogen auf eingesetztes Acetal.

Beispiel 8

Man arbeitet wie in Beispiel 1 angegeben, jedoch unter Zusatz von 0,57 g p-Toluolsulfonsäure und 1,44 g Di-2-ethylhexylamin, und erhält 769,9 g Destillat.

Die Ausbeute an Ethyl-1-propenylether beträgt 97,6 % der Theorie, bezogen auf eingesetztes Acetal.

Beispiel 9

Man arbeitet wie in Beispiel 1 angegeben, jedoch unter Zusatz von 0,57 g p-Toluolsulfonsäure und 0,68 g Isononylamin (hergestellt durch Hydroformylierung von Diisobutylen und nachfolgender reduktiver Aminierung), und erhält 773,8 g Destillat.

Die Ausbeute an Ethyl-1-propenylether beträgt 98,1 % der Theorie, bezogen auf eingesetztes Acetal.

Beispiel 10

Man arbeitet wie in Beispiel 1 angegeben, jedoch unter Zusatz von 0,57 g p-Toluolsulfonsäure und 1,28 g Diisononylamin (hergestellt durch Hydroformylierung von Diisobutylen und nachfolgender reduktiver Aminierung), und erhält 708,3 g Destillat.

Die Ausbeute an Ethyl-1-propenylether beträgt 97,1 % der Theorie, bezogen auf eingesetztes Acetal.

6

Beispiel 11

Man arbeitet wie in Beispiel 1 angegeben, jedoch unter Zusatz von 0,57 g p-Toluolsulfonsäure und 0,75 g Diamylamin, und erhält 771,5 g Destillat.

Die Ausbeute an Ethyl-1-propenylether beträgt 97,8 % der Theorie, bezogen auf eingesetztes Acetal.

Beispiel 12

Man arbeitet wie in Beispiel 1 angegeben, jedoch unter Zusatz von 0,28 g p-Toluolsulfonsäure und 0,44 g Tri-n-butylamin, und erhält 765,3 g Destillat.

Die Ausbeute an Ethyl-1-propenylether beträgt 96,9 % der Theorie, bezogen auf eingesetztes Acetal.

Die Zusammensetzung des in den vorangegangenen Beispielen jeweils erhaltenen Destillats (gaschromatografisch bestimmt) ist der nachfolgenden Tabelle 1 zu entnehmen.

Tabelle

| Beispiel | 1 | 2 | 3 | 4 | 5 | 6 | 7 | 8 | 9 | 10 | 11 | 12 | |
|---|---|---|---|---|---|---|---|---|---|---|---|---|---|
| Vergleichs-beispiel | | | | | | | | | | | | | 1 |
| Zusammen-setzung* (Gew.-%) | | | | | | | | | | | | | |
| Ethanol | 33,7 | 33,6 | 30,3 | 32,7 | 32,1 | 33,7 | 32,3 | 33,7 | 33,7 | 33,6 | 33,7 | 33,7 | 12,5 |
| Propanal | 0,2 | 0,2 | 0,2 | 1,4 | 1,5 | 0,2 | 0,3 | 0,2 | 0,2 | 0,2 | 0,2 | 0,2 | 0,2 |
| Cyclohexan | 3,9 | 4,0 | 4,1 | 0,6 | 0,7 | 3,9 | 3,8 | 3,8 | 3,7 | 3,9 | 3,8 | 3,7 | 4,1 |
| Ethyl-1-propenylether | 61,9 | 61,7 | 55,4 | 61,0 | 60,1 | 61,9 | 59,3 | 62,0 | 62,0 | 61,8 | 62,0 | 61,9 | 22,2 |
| Propanal-diethylacetal | 0,1 | 0,3 | 9,5 | 2,9 | 3,8 | 0,1 | 3,8 | 0,1 | 0,1 | 0,1 | 0,1 | 0,1 | 59,9 |
| sonstige Komponenten | 0,2 | 0,2 | 0,5 | 1,4 | 1,8 | 0,2 | 0,5 | 0,2 | 0,3 | 0,4 | 0,2 | 0,4 | 1,1 |
| Destillat (g) | 775,4 | 760,5 | 754,5 | 758,5 | 735,5 | 772,7 | 755,4 | 769,9 | 773,8 | 708,3 | 771,5 | 765,3 | 748,5 |
| Ausbeute** (% d.Th.) | 98,2 | 95,9 | 85,5 | 93,2 | 89,1 | 97,8 | 91,7 | 97,6 | 98,1 | 97,1 | 97,8 | 96,9 | 34,0 |

\* Zusammensetzung durch gaschromatografische Analyse ermittelt
\*\* bezogen auf eingesetztes Diacetal

EP 0 490 221 B1

IIb) Herstellung von Butyl-1-butenylether

Beispiel 13

In einem mit einer Kolonne (24 theoretische Böden) bestückten 2 l Rundkolben werden 800 g n-Butyraldehyd-di-n-butylacetal (hergestellt wie unter (B) beschrieben), 0,19 g p-Toluolsulfonsäure und 0,38 g Di-2-ethylhexylamin vorgelegt und erhitzt. Man destilliert die infolge der Acetalspaltung freiwerdenden Reaktionsprodukte (n-Butanol und Butyl-1-butenylether) unter einem Rücklaufverhältnis von 0,5 : 1 und einem Druck von 200 mbar ab. Die Temperatur am Kopf der Kolonne beträgt 74 bis 81°C. Die Destillation wird beendet, sobald eine Sumpftemperatur von 200°C erreicht wird. Man erhält 770,2 g Destillat folgender Zusammensetzung (gaschromatografisch ermittelt):
37,4 Gew.-% n-Butanol
0,1 Gew.-% n-Butyraldehyd
0,1 Gew.-% Cyclohexan
60,3 Gew.-% Butyl-1-butenylether
1,1 Gew.-% n-Butyraldehyd-di-n-butylacetal
1,0 Gew.-% sonstige Komponenten
Die Ausbeute an Butyl-1-butenylether beträgt 95,8 % der Theorie, bezogen auf eingesetztes Acetal.

Vergleichsbeispiel 2

Man arbeitet wie in Beispiel 13 angegeben, jedoch ohne Zusatz von Di-2-ethylhexylamin, und erhält 763,1 g Destillat folgender Zusammensetzung (gaschromatografisch ermittelt):
23,1 Gew.-% n-Butanol
0,1 Gew.-% Cyclohexan
35,5 Gew.-% Butyl-1-butenylether
39,8 Gew.-% n-Butyraldehyd-di-n-butylacetal
1,5 Gew.-% sonstige Komponenten
Die Ausbeute an Butyl-1-butenylether beträgt 55,8 % der Theorie, bezogen auf eingesetztes Acetal.

Beispiel 14

Man arbeitet wie in Beispiel 13 angegeben, jedoch unter Zusatz von 0,38 g p-Toluolsulfonsäure und 0,76 g Di-2-ethylhexylamin, und erhält 778,2 g Destillat folgender Zusammensetzung (gaschromatografisch ermittelt):
37,8 Gew.-% n-Butanol
0,1 Gew.-% n-Butyraldehyd
0,1 Gew.-% Cyclohexan
61,3 Gew.-% Butyl-1-butenylether
0,2 Gew.-% n-Butyraldehyd-di-n-butylacetal
0,5 Gew.-% sonstige Komponenten
Die Ausbeute an Butyl-1-butenylether beträgt 98,3 % der Theorie, bezogen auf eingesetztes Acetal.

IIc) Herstellung von 3-Methyl-1-butenylpropylether

Beispiel 15

In einem mit einer Kolonne (24 theoretische Böden) bestückten 2 l Rundkolben werden 800 g 3-Methylbutyraldehyd-di-n-propylacetal (hergestellt wie unter (C) beschrieben), 0,2 g p-Toluolsulfonsäure und 0,39 g Di-2-ethylhexylamin vorgelegt und erhitzt. Man destilliert die infolge der Acetalspaltung freiwerdenden Reaktionsprodukte (n-Propanol und 3-Methyl-1-butenylpropylether) unter einem Rücklaufverhältnis von 1 : 1 und einem Druck von 200 mbar ab. Die Temperatur am Kopf der Kolonne beträgt 60 bis 63°C. Die Destillation wird beendet, sobald eine Sumpftemperatur von 200°C erreicht wird. Man erhält 775,6 g Destillat folgender Zusammensetzung (gaschromatografisch ermittelt):
34,7 Gew.-% n-Propanol
0,2 Gew.-% 3-Methylbutyraldehyd
0,2 Gew.-% Cyclohexan
63,9 Gew.-% 3-Methyl-1-butenylpropylether

0,2 Gew.-% 3-Methylbutyraldehyd-di-n-propylacetal
0,8 Gew.-% sonstige Komponenten
Die Ausbeute an 3-Methyl-1-butenylpropylether beträgt 99,0 % der Theorie, bezogen auf eingesetztes Acetal.

Vergleichsbeispiel 3

Man arbeitet wie in Beispiel 15 angegeben, jedoch ohne Zusatz von Di-2-ethylhexylamin, und erhält 765,5 g Destillat folgender Zusammensetzung (gaschromatografisch ermittelt):
32,0 Gew.-% n-Propanol
0,3 Gew.-% 3-Methylbutyraldehyd
0,2 Gew.-% Cyclohexan
58,2 Gew.-% 3-Methyl-1-butenyl-propylether
6,5 Gew.-% 3-Methylbutyraldehyd-di-n-propylacetal
2,8 Gew.-% sonstige Komponenten
Die Ausbeute an 3-Methyl-1-butenylpropylether beträgt 89,0 % der Theorie, bezogen auf eingesetztes Acetal.

IId) Herstellung von 2-Ethyl-1-butenylmethylether

Beispiel 16

In einem mit einer Kolonne (24 theoretische Böden) bestückten 2 l Rundkolben werden 720 g 2-Ethylbutyraldehyd-dimethylacetal (hergestellt wie unter (D) beschrieben), 0,48 g p-Toluolsulfonsäure und 0,96 g 2-Ethylhexylamin vorgelegt und erhitzt. Man destilliert die infolge der Acetalspaltung freiwerdenden Reaktionsprodukte (Methanol und 2-Ethyl-1-butenylmethylether) unter einem Rücklaufverhältnis von 10 : 1 und einem Druck von 1013 mbar ab. Die Temperatur am Kopf der Kolonne beträgt 104 bis 115°C. Die Destillation wird beendet, sobald eine Sumpftemperatur von 200°C erreicht wird.
Man erhält 692,5 g Destillat folgender Zusammensetzung (gaschromatografisch ermittelt):
21,4 Gew.-% Methanol
0,1 Gew.-% Cyclohexan
0,4 Gew.-% Ethylbutyraldehyd
77,1 Gew.-% 2-Ethyl-1-butenylmethylether
0,1 Gew.-% 2-Ethylbutyraldehyd-dimethylacetal
0,9 Gew.-% sonstige Komponenten
Die Ausbeute an 2-Ethyl-1-butenylmethylether beträgt 97,0 % der Theorie, bezogen auf eingesetztes Acetal.

Vergleichsbeispiel 4

Man arbeitet wie in Beispiel 16 angegeben, jedoch mit 0,48 g p-Toluolsulfonsäure, aber ohne Zusatz von 2-Ethylhexylamin, und erhält 683,2 g Destillat folgender Zusammensetzung (gaschromatografisch ermittelt):
19,6 Gew.-% Methanol
0,1 Gew.-% Cyclohexan
0,4 Gew.-% 2-Ethylbutyraldehyd
70,1 Gew.-% 2-Ethyl-1-butenylmethylether
8,1 Gew.% 2-Ethylbutyraldehyd-dimethylacetal
1,7 Gew.-% sonstige Komponenten
Die Ausbeute an 2-Ethyl-1-butenylmethylether beträgt 87,0 % der Theorie, bezogen auf eingesetztes Acetal.

**Patentansprüche**

1. Verfahren zur Herstellung von Ethern der Formel $R^1\text{-}CR^2=CH\text{-}O\text{-}R^3$, worin $R^1$ für einen Rest mit 1 bis 6 Kohlenstoffatomen, $R^2$ für Wasserstoff oder einen Rest mit 1 bis 2 Kohlenstoffatomen und $R^3$ für einen Rest mit 1 bis 6 Kohlenstoffatomen steht, dadurch gekennzeichnet, daß ein Diacetal der Formel $R^1\text{-}CHR^2\text{-}CH(\text{-}O\text{-}R^3)_2$ in Gegenwart eines aus einer Säure und einem aliphatischen oder cycloaliphatischen Amin bestehenden Katalysators bei erhöhter Temperatur umgesetzt wird.

2. Verfahren nach Anspruch 1 dadurch gekennzeichnet, daß je Mol Diacetal 0,00015 bis 0,008, insbesondere 0,0002 bis 0,006, bevorzugt 0,00025 bis 0,0015 Mol Säure eingesetzt werden.

3. Verfahren nach Anspruch 1 oder 2, dadurch gekennzeichnet, daß Säure zu Amin im molaren Verhältnis von 1 : 0,7 bis 1 : 3,5, insbesondere 1 : 0,8 bis 1 : 3, bevorzugt 1 : 1 bis 1 : 2 eingesetzt werden.

4. Verfahren nach einem oder mehreren der Ansprüche 1 bis 3, dadurch gekennzeichnet, daß die Säure einen pK-Wert $\leq$ 2,5, insbesondere $\leq$ 2,2, bevorzugt $\leq$ 2,0 aufweist.

5. Verfahren nach einem oder mehreren der Ansprüche 1 bis 4, dadurch gekennzeichnet, daß als Säure Phosphorsäure, Schwefelsäure, eine aliphatische oder aromatische Sulfonsäure, insbesondere eine aromatische Sulfonsäure eingesetzt wird.

6. Verfahren nach einem oder mehreren der Ansprüche 1 bis 5, dadurch gekennzeichnet, daß als Säure p-Toluolsulfonsäure eingesetzt wird.

7. Verfahren nach einem oder mehreren der Ansprüche 1 bis 6, dadurch gekennzeichnet, daß als Amin ein Amin mit insgesamt 8 bis 30, insbesondere 9 bis 24, bevorzugt 10 bis 20 Kohlenstoffatomen eingesetzt wird.

8. Verfahren nach einem oder mehreren der Ansprüche 1 bis 7, dadurch gekennzeichnet, daß als Amin ein aliphatisches geradkettiges und/oder verzweigtes Amin, insbesondere n-Hexyl-, n-Heptyl-, n-Octyl-, n-Decyl-, n-Dodecyl-, 2-Ethylhexyl-, i-Nonyl-, 3,5,5-Trimethylhexyl-, Di-n-butyl-, Di-i-butyl-, Diamyl-, Di-n-hexyl-, Di-n-octyl-, Di-2-ethylhexyl-, Di-i-nonyl-, Tri-n-propyl-, Tri-n-butyl-, Tri-n-pentyl-, Tri-n-hexyl-, Tri-n-octyl-, Tri-2-ethylhexyl-, Tri-n-nonyl-, Tri-i-nonyl- und Tri-n-decylamin eingesetzt.

9. Verfahren nach einem oder mehreren der Ansprüche 1 bis 8, dadurch gekennzeichnet, daß die Umsetzung bei einer Temperatur oberhalb der Siedetemperatur der sich bildenden Reaktionsprodukte durchgeführt wird.

10. Verfahren nach einem oder mehreren der Ansprüche 1 bis 9, dadurch gekennzeichnet, daß die Temperatur höchstens 200, insbesondere 180, bevorzugt 160 °C beträgt.

**Claims**

1. A process for preparing ethers of the formula $R^1\text{-}CR^2 = CH\text{-}O\text{-}R^3$, in which $R^1$ is a radical having 1 to 6 carbon atoms, $R^2$ is hydrogen or a radical having 1 to 2 carbon atoms and $R^3$ is a radical having 1 to 6 carbon atoms, characterised in that a diacetal of the formula $R^1\text{-}CHR^2\text{-}CH(\text{-}O\text{-}R^3)_2$ is reacted at elevated temperature in the presence of a catalyst consisting of an acid and an aliphatic or cycloaliphatic amine.

2. A process according to claim 1, characterised in that 0.00015 to 0.008, in particular 0.0002 to 0.006, preferably 0.00025 to 0.0015 mol of acid is employed per mole of diacetal.

3. A process according to claim 1 or 2, characterised in that acid and amine are employed in an acid to amine molar ratio of 1 : 0.7 to 1 : 3.5, in particular 1 : 0.8 to 1 : 3, preferably 1 : 1 to 1 : 2.

4. A process according to one or more of claims 1 to 3, characterised in that the acid has a pK of $\leq$ 2.5, in particular $\leq$ 2.2, preferably $\leq$ 2.0.

5. A process according to one or more of claims 1 to 4, characterised in that the acid employed is phosphoric acid, sulfuric acid, or an aliphatic or aromatic sulfonic acid, in particular an aromatic sulfonic acid.

6. A process according to one or more of claims 1 to 5, characterised in that the acid employed is p-toluenesulphonic acid.

7. A process according to one or more of claims 1 to 6, characterised in that the amine employed is an amine having a total of 8 to 30, in particular 9 to 24, preferably 10 to 20 carbon atoms.

8. A process according to one or more of claims 1 to 7, characterised in that the amine employed is an aliphatic straight-chain and/or branched amine, in particular n-hexyl-, n-heptyl-, n-octyl-, n-decyl-, n-dodecyl-, 2-ethylhexyl-, isononyl-, 3,5,5-trimethylhexyl-, di-n-butyl-, di-isobutyl-, diamyl-, di-n-hexyl-, di-n-octyl-, di-2-ethylhexyl-, di-isononyl-, tri-n-propyl-, tri-n-butyl-, tri-n-pentyl-, tri-n-hexyl-, tri-isononyl-, tri-n-octyl-, tri-2-ethylhexyl-, tri-n-nonyl- and tri-n-decylamine.

9. A process according to one or more of claims 1 to 8, characterised in that the reaction is carried out at a reaction temperature above the boiling temperature of the reaction products formed.

10. A process according to one or more of claims 1 to 9, characterised in that the temperature is not more than 200, in particular 180, preferably 160 °C.

**Revendications**

1. Procédé de préparation d'éthers-oxydes de formule $R^1\text{-}CR^2 = CH\text{-}O\text{-}R^3$, dans laquelle $R^1$ représente un reste comportant 1 à 6 atomes de carbone, $R^2$ représente un atome d'hydrogène ou un reste comportant 1 à 2 atomes de carbone et $R^3$ représente un reste comportant 1 à 6 atomes de carbone, procédé caractérisé en ce qu'on fait réagir à température élevée un diacétal de formule $R^1\text{-}CHR^2(\text{-}O\text{-}R^3)_2$ en présence d'un catalyseur consistant en un acide et en une amine aliphatique ou cycloaliphatique.

2. Procédé selon la revendication 1, caractérisé en ce qu'on utilise par mole de diacétal 0,00015 à 0,008, notamment 0,0002 à 0,006, de préférence 0,00025 à 0,015 mol d'acide.

3. Procédé selon la revendication 1 ou 2, caractérisé en ce qu'on utilise l'acide par rapport à l'amine en un rapport molaire de 1:0,7 à 1:3,5, notamment de 1:0,8 à 1:3 et de préférence de 1:1 à 1:2.

4. Procédé selon une ou plusieurs des revendications 1 à 3, caractérisé en ce que l'acide présente une valeur de pK $\leq$ 2,5, notamment $\leq$ 2,2 et de préférence $\leq$ 2,0.

5. Procédé selon une ou plusieurs des revendications 1 à 4, caractérisé en ce qu'on utilise comme acide l'acide phosphorique, l'acide sulfurique, un acide sulfonique aliphatique ou aromatique, notamment un acide sulfonique aromatique.

6. Procédé selon une ou plusieurs des revendications 1 à 5, caractérisé en ce qu'on utilise comme acide l'acide p-toluènesulfonique.

7. Procédé selon une ou plusieurs des revendications 1 à 6, caractérisé en ce qu'on utilise comme amine une amine comportant au total 8 à 30, notamment 9 à 24 et de préférence 10 à 20 atomes de carbone.

8. Procédé selon une ou plusieurs des revendications 1 à 7, caractérisé en ce qu'on utilise comme amine une amine aliphatique, linéaire et/ou ramifiée, notamment la n-hexylamine, la n-heptylamine, la n-octylamine, la n-décylamine, la n-dodécylamine, la 2-éthylhexylamine, l'i-nonylamine, la 3,5,5-tri-méthylhexylamine, la di-n-butylamine, la di-i-butylamine, la diamylamine, la di-n-hexylamine, la di-n-octylamine, la di-2-éthylhexylamine, la di-i-nonylamine, la tri-n-propylamine, la tri-n-butylamine, la tri-n-pentylamine, la tri-n-hexylamine, la tri-n-octylamine, la tri-2-éthylhexylamine, la tri-n-nonylamine, la tri-iso-nonylamine et la tri-n-décylamine.

9. Procédé selon une ou plusieurs des revendications 1 à 8, caractérisé en ce qu'on conduit la réaction à une température supérieure à la température d'ébullition des produits de réaction qui se forment.

10. Procédé selon une ou plusieurs des revendications 1 à 9, caractérisé en ce que la température vaut au maximum 200, en particulier 180 et de préférence 160 °C.